# EUROPEAN PATENT APPLICATION

(11) **EP 4 563 700 A1**
(43) Date of publication of application: **04.06.2025**
(21) Application number: 23853009.1
(22) Date of filing: 10.08.2023
(51) Int. Cl.: C12N 9/10, C12N 15/77, C12P 13/08

(54) **NOVEL ACETOHYDROXY ACID SYNTHASE SUBUNIT VARIANT AND METHOD FOR PRODUCING L-VALINE USING SAME**

(30) Priority: 10.08.2022 KR 20220099926
(71) Applicant: CJ Cheiljedang Corporation, Seoul 04560 (KR)
(72) Inventor: LEE, Jungseok, Seoul 04560 (KR); KIM, Seon Hye, Seoul 04560 (KR); JU, Si-Yeon, Seoul 04560 (KR); LEE, Sung Gun, Seoul 04560 (KR); CHOI, Jin-Geun, Seoul 04560 (KR); JUNG, Byung Kwon, Seoul 04560 (KR)
(74) Representative: Meissner Bolte Partnerschaft mbB
(86) International application number: PCT/KR2023/011801
(87) International publication number: WO 2024/035140

(57) **Abstract**

The present disclosure relates to a novel acetohydroxy acid synthase subunit (ilvN) variant, a polynucleotide encoding the variant of the present disclosure, an L-valine-producing microorganism comprising the acetohydroxy acid synthase subunit (ilvN) variant of the present disclosure, and a method for producing L-valine using the microorganism of the present disclosure.

## Description

### [Technical Field]

The present disclosure relates to a novel acetohydroxy acid synthase subunit (ilvN) variant, a polynucleotide encoding the variant of the present disclosure, an L-valine-producing microorganism comprising the acetohydroxy acid synthase subunit (ilvN) variant of the present disclosure, and a method for producing L-valine using the microorganism of the present disclosure.

### [Background Art]

L-Amino acids are basic structural units of proteins and used as important materials for pharmaceuticals, food additives, animal feeds, nutrients, pesticides, bactericides, and the like. In particular, branched-chain amino acid (BCAA) is a generic term for L-valine, L-leucine, and L-isoleucine, which are essential amino acids, and the branched-chain amino acids are known to have an antioxidant effect and an effect of promoting protein synthesis in muscle cells.

Meanwhile, the production of branched-chain amino acids using microorganisms are mainly achieved by the microorganisms of the genus *Corynebacterium* and are known to be biosynthesized from 2-ketoisocaproate as a precursor after undergoing several steps from pyruvic acid [Korean Patent Nos. 10-0220018 and 10-0438146]. However, the production of L-branched-chain amino acids by these microorganisms poses challenges in achieving large-scale industrial production.

Under these circumstances, the present inventors have confirmed that the introduction of a variant with enhanced activity of the gene *ilvN* (an acetohydroxy acid synthase small subunit), which encodes an enzyme involved in the microbial L-valine biosynthesis, into a microorganism for the purpose of improving L-valine-producing ability using the microorganism significantly increases the L-valine producing ability of the microorganism.

### [Disclosure]

### [Technical Problem]

The purpose of the present disclosure is to provide a novel acetohydroxy acid synthase subunit (ilvN) variant, a polynucleotide encoding the variant of the present disclosure, an L-valine-producing microorganism comprising the acetohydroxy acid synthase subunit (ilvN) variant of the present disclosure, and a method for producing L-valine using the microorganism of the present disclosure.

### [Technical Solution]

An object of the present disclosure is to provide an acetohydroxy acid synthase subunit (ilvN) variant, in which an amino acid corresponding to position 159 in an amino acid sequence of SEQ ID NO: 1 is substituted with another amino acid.

Another object of the present disclosure is to provide a polynucleotide encoding the variant of the present disclosure.

Still another object of the present disclosure is to provide a microorganism comprising the variant of the present disclosure or a polynucleotide encoding the variant of the present disclosure.

Still another object of the present disclosure is to provide a method for producing L-valine, comprising culturing the microorganism in a medium.

Still another object of the present disclosure is to provide a composition for producing L-valine, which comprises: the microorganism of the present disclosure; the culture medium obtained by culturing the microorganism of the present disclosure; or a combination of thereof.

### [Advantageous Effects]

Culturing the microorganism comprising an acetohydroxy acid synthase subunit variant of the present disclosure enables a high-yield production of L-valine compared with a microorganism having a conventional, non-modified polypeptide.

### [Detailed Description of Preferred Embodiments]

The present disclosure will be described in detail as follows. Meanwhile, each description and embodiment disclosed in the present disclosure may also be applied to each of other descriptions and embodiments. That is, all combinations of various elements disclosed in the present disclosure fall within the scope of the present disclosure. Further, the scope of the present disclosure is not limited by the specific description described below. Furthermore, a number of papers and patent documents are referenced and cited throughout this specification. The disclosures of the cited papers and patent documents are incorporated herein by reference in their entirety to further clarify the level of art and the description of the present disclosure.

An aspect of the present disclosure provides an acetohydroxy acid synthase subunit (ilvN) variant in which an amino acid corresponding to position 159 in an amino acid sequence of SEQ ID NO: 1 is substituted with another amino acid.

As used herein, the term "acetohydroxy acid synthase subunit (ilvN) variant" refers to a variant of the acetohydroxy acid synthase subunit (ilvN), which includes one or more amino acid substitutions in the amino acid sequence of a polypeptide having acetohydroxy acid synthase subunit (ilvN) activity.

As used herein, the term "acetohydroxy acid synthase", which is the first enzyme involved in the biosynthesis of L-valine, is also referred to as acetolactate synthase. Acetohydroxy acid synthase can produce acetolactate (i.e., a precursor of valine) by catalyzing the decarboxylation of pyruvate and a condensation reaction with another pyruvate molecule , or produce acetohydroxybutyrate *(i.e.,* a precursor of isoleucine) by catalyzing the decarboxylation of pyruvate and a condensation reaction with 2-ketobutyrate.

Acetohydroxy acid synthase is encoded by two genes, *ilvB* and *ilvN.* **The** *ilvB* gene encodes the large subunit of acetohydroxy acid synthase, and the *ilvN* gene encodes the small subunit of acetohydroxy acid synthase. Between the two subunits, the small subunit encoded by the *ilvN* gene is considered to play a critical role in feedback inhibition.

As used herein, the term "acetohydroxy acid synthase subunit (ilvN)" may be an acetohydroxy acid synthase subunit (ilvN) encoded by the *ilvN* gene, but is not particularly limited thereto. Further, the acetohydroxy acid synthase subunit (ilvN) may be an acetohydroxy acid synthase subunit (ilvN) protein derived from a microorganism of the genus *Corynebacterium,* or specifically *Corynebacterium glutamicum,* or a variant thereof, but is not limited thereto. Specifically, the acetohydroxy acid synthase subunit (ilvN) protein may comprise, for example, the amino acid sequence of SEQ ID NO: 1 or an amino acid sequence having 70% or more homology or identity therewith, but is not limited thereto as long as it retains the activity of the acetohydroxy acid synthase subunit (ilvN). Specifically, the amino acid sequence may comprise the amino acid sequence of SEQ ID NO: 1 or an amino acid sequence having at least 70%, 80%, 85%, 90%, 95%, 96%, 97%, 98%, or 99% or more homology or identity with the amino acid sequence of SEQ ID NO: 1. The sequence of SEQ ID NO: 1 may be obtained from publicly accessible databases, such as the Genbank of NCBI or Kyoto Encyclopedia of Genes and Genomes (KEGG). For example, the amino acid sequence may be derived from a microorganism of the genus *Corynebacterium,* or specifically *Corynebacterium glutamicum,* and more specifically, it may be a polypeptide or protein comprising the amino acid sequence of SEQ ID NO: 1, but is not limited thereto. Further, it is obvious that any auxiliary protein having an amino acid sequence with deletions, modifications, substitutions, or additions in a part thereof may also be included within the scope of the present disclosure as long as the amino acid sequence has such homology or identity and exhibits equivalent efficacy to that of the protein.

Further, the acetohydroxy acid synthase subunit (ilvN) protein having the amino acid sequence of SEQ ID NO: 1 may have or comprise the sequence of SEQ ID NO: 2 or a nucleotide sequence having 70% or more, 75% or more, 80% or more, 85% or more, 90% or more, 95% or more, 96% or more, 97% or more, or 98% or more, and less than 100% homology or identity with the sequence of SEQ ID NO: 2, or may be encoded by a polynucleotide consisting of or essentially consisting of the sequence of SEQ ID NO: 2 or a nucleotide sequence having 70% or more, 75% or more, 80% or more, 85% or more, 90% or more, 95% or more, 96% or more, 97% or more, or 98% or more, and less than 100% homology or identity with the sequence of SEQ ID NO: 2, but is not limited thereto.

As used herein, the term "variant" refers to a polypeptide in which the amino acid sequence is different due to conservative substitutions and/or modifications of one or more amino acids from the amino acid sequence of the variant before mutation but maintains its functions or properties before mutation. Such variants may generally be identified by modifying one or more amino acids in the amino acid sequence of the polypeptide and evaluating properties of the modified polypeptide. That is, the ability of the variant may be increased, unchanged, or reduced compared with that of the polypeptide before mutation. Additionally, some variants may include those in which at least one part, such as an N-terminal leader sequence or a transmembrane domain, is removed. Other variants may be those in which part of the N-terminus and/or C-terminus of a mature protein is removed. The term "variant" may be used interchangeably with "modification", "modified polypeptide", "modified protein", "mutant", "mutein", "divergent", or the like, and any term that is used in a sense of being mutated may be used without limitation.

Additionally, the variant may include deletions or additions of amino acids that have a minimal effect on the properties and secondary structure of a polypeptide. For example, the polypeptide may be conjugated to a signal (or leader) sequence of the N-terminal of a protein involved in the transfer of the protein co-translationally or post-translationally. Further, the polypeptide may also be conjugated to another sequence or linker to identify, purify, or synthesize the polypeptide.

The acetohydroxy acid synthase subunit (ilvN) variant of the present disclosure may be an acetohydroxy acid synthase subunit (ilvN) variant in which the amino acid corresponding to position 159 in an amino acid sequence of SEQ ID NO: 1 is substituted with another amino acid, but is not limited thereto.

In one embodiment, the acetohydroxy acid synthase subunit (ilvN) variant may further have the amino acid corresponding to position 42 in the amino acid sequence of SEQ ID NO: 1 substituted with another amino acid, in addition to the substitution of the amino acid corresponding to position 159 in an amino acid sequence of SEQ ID NO: 1 with another amino acid

The "another amino acid" or "other amino acids" is not limited as long as the amino acids differ from the amino acid before substitution. Meanwhile, it is obvious that the expression "a specific amino acid is substituted" implies that the amino acid is substituted with an amino acid different from the amino acid before the substitution, even if it is not explicitly described such that the amino acid is substituted with a different amino acid.

Amino acids may generally be categorized based on the similarity in polarity, charge, solubility, hydrophobicity, hydrophilicity, and/or amphipathic nature of the residues.

For example, positively charged (basic) amino acids may include arginine, lysine, and histidine; negatively charged (acidic) amino acids include glutamate and aspartate; amino acids with nonpolar side chains (nonpolar amino acids) may include glycine, alanine, valine, leucine, isoleucine, methionine, phenylalanine, tryptophan, and proline; and amino acids with polar or hydrophilic side chains (polar amino acids) include serine, threonine, cysteine, tyrosine, asparagine, and glutamine. **In** another example, amino acids with electrically charged side chains (electrically charged amino acids) include arginine, lysine, histidine, glutamate, and aspartate, and amino acids with electrically uncharged side chains (also referred to as uncharged amino acids or neutral amino acids) include glycine, alanine, valine, leucine, isoleucine, methionine, phenylalanine, tryptophan, proline, serine, threonine, cysteine, tyrosine, asparagine, and glutamine. In another example, phenylalanine, tryptophan, and tyrosine may be categorized into aromatic amino acids. In another example, valine, leucine, and isoleucine may be categorized into branched amino acids. In another example, 20 types of amino acids may be classified into five groups by size, starting from the group of amino acids with relatively small volumes: glycine, alanine, and serine; cysteine, proline, threonine, aspartate, and asparagine; valine, histidine, glutamate, and glutamine; isoleucine, leucine, methionine, leucine, and arginine; and phenylalanine, tryptophan, and tyrosine. However, the classification of amino acids is not limited thereto.

For example, the phrase "the amino acid corresponding to position 159 in SEQ ID NO: 1 is substituted with another amino acid" may mean that the amino acid is substituted with glutamate, phenylalanine, glycine, arginine, aspartate, cysteine, asparagine, glutamine, histidine, proline, serine, tyrosine, isoleucine, lysine, tryptophan, valine, methionine, threonine, or leucine, excluding alanine, and the phrase "the amino acid corresponding to position 42 in SEQ ID NO: 1 is substituted with another amino acid" may mean that the amino acid is substituted with valine, asparagine, glycine, arginine, aspartate, cysteine, glutamate, histidine, proline, serine, tyrosine, isoleucine, leucine, lysine, phenylalanine, tryptophan, glutamine, methionine, or threonine, excluding alanine, but is not limited thereto.

When the expression "a protein having the amino acid sequence of a specific SEQ ID NO" is used in the present disclosure, it is obvious that a protein having an amino acid sequence with deletions, modifications, substitutions, conservative substitutions, or additions in part thereof may also be used in the present disclosure, provided that the protein exhibits identical or equivalent activity to the protein consisting of the amino acid sequence of the corresponding SEQ ID NO. For example, when exhibiting identical or equivalent activity to the variant protein, it is obvious that a protein with sequence additions, naturally-occurring mutations, or silent mutations, or conservative substitutions thereof that do not alter the protein function is not excluded, and that such proteins having sequence additions or mutations fall within the scope of the present disclosure.

As used herein, the term "position N" may include the Nth position and the amino acid positions corresponding to the Nth position. Specifically, the term may include the amino acid position corresponding to an arbitrary amino acid residue in a mature polypeptide of a specific amino acid sequence. The specific amino acid sequence may be the amino acid sequence of SEQ ID NO: 1.

As used herein, the term "corresponding to" refers to an amino acid residue at a listed position in a polypeptide or an amino acid residue that is similar, identical, or homologous to the listed amino acid residue in the polypeptide. Identifying the amino acid at the corresponding position may be determining a specific amino acid in a sequence that refers to a specific sequence. As used herein, the term "corresponding region" generally refers to a similar or corresponding position in a related protein or reference protein.

For example, an arbitrary amino acid sequence may be aligned with SEQ ID NO: 1, and based thereon, each amino acid residue of the arbitrary amino acid sequence may be numbered with reference to the numerical positions of amino acid residues corresponding to the amino acid residues of SEQ ID NO: 1. For example, a sequence alignment algorithm as described in the present disclosure may determine the position of an amino acid or a position at which modification such as substitution, insertion, or deletion occurs through comparison with a query sequence (also referred to as a "reference sequence").

In such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000, Trends Genet. 16: 276-277), *etc.* may be used, but are not limited thereto, and a sequence alignment program, a pairwise sequence comparison algorithm, *etc.,* which are known in the art, may be appropriately used.

In one embodiment, the acetohydroxy acid synthase subunit (ilvN) variant of the present disclosure may include an amino acid sequence in which one or two of the amino acids corresponding to positions 42 and 159 from the N-terminus in SEQ ID NO: 1 are substituted with amino acids different amino acids.

In one embodiment of the previously described embodiments, the acetohydroxy acid synthase subunit (ilvN) variant of the present disclosure may be a polypeptide in which the amino acid corresponding to position 159 in SEQ ID NO: 1 (alanine) is substituted with glutamate, but is not limited thereto.

In one embodiment of the previously described embodiments, the acetohydroxy acid synthase subunit (ilvN) variant of the present disclosure may be a polypeptide in which the amino acid corresponding to position 159 in SEQ ID NO: 1 (alanine) is substituted with glutamate and the amino acid corresponding to position 42 in SEQ ID NO: 1 (alanine) is substituted with valine, but is not limited thereto.

In one embodiment of the previously described embodiments, the variant provided in the present disclosure may include substitution of the amino acid corresponding to position 159 from the N-terminus in SEQ ID NO: 1 with another amino acid.

In one embodiment of the previously described embodiments, the variant provided in the present disclosure may include substitution of the amino acid corresponding to position 159 from the N-terminus in SEQ ID NO: 1 with an amino acid selected from arginine, lysine, histidine, glutamate, and aspartate, all of which are amino acids with electrically charged side chains. For example, the amino acid may be selected from glutamate and aspartate, which are acidic amino acids. In one embodiment of the previously described embodiments, the variant may be a variant in which the amino acid corresponding to position 159 from the N-terminus in SEQ ID NO: 1 is substituted with glutamate (E).

In one embodiment of the previously described embodiments, the variant provided in the present disclosure may include substitution of the amino acid corresponding to position 42 from the N-terminus in SEQ ID NO: 1 with another amino acid.

In one embodiment of the previously described embodiments, the variant may have substitution of the amino acid corresponding to position 42 from the N-terminus in SEQ ID NO: 1 with a nonpolar amino acid. For example, the nonpolar amino acid may be selected from glycine, alanine, valine, leucine, isoleucine, methionine, phenylalanine, tryptophan, and proline. In one embodiment of the previously described embodiments, the variant may be a variant in which the amino acid corresponding to position 42 from the N-terminus in SEQ ID NO: 1 is substituted with valine (V).

Meanwhile, a person of ordinary skill in the art can identify the amino acids that correspond to positions 159 and 42 in the amino acid sequence of SEQ ID NO: 1 of the present disclosure through a sequence alignment known in the art. Further, it is obvious that the expression "an amino acid at a specific position in a specific SEQ ID NO" also includes "the amino acid corresponding to the specific position" in any amino acid sequence even if the present disclosure does not explicitly describe the same.

Further, the variant of the present disclosure may include an amino acid sequence which has at least 70%, 75%, 80%, 85%, 90%, 95%, 96%, 97%, 98%, 99%, 99.5%, 99.7%, or 99.9% or more homology or identity with the amino acid sequence of SEQ ID NO: 1 in which the amino acid corresponding to position 159 from the N-terminus in SEQ ID NO: 1 is substituted with another amino acid, or include an amino acid sequence of SEQ ID NO: 1 in which the amino acid corresponding to position 42 is substituted with another amino acid in addition to the amino acid corresponding to position 159 being substituted with another amino acid. Further, it is obvious that a variant having an amino acid sequence with deletions, modifications, substitutions, conservative substitutions, or additions in part thereof falls within the scope of the present disclosure as long as the amino acid sequence has such homology or identity and exhibits equivalent efficacy as the variant of the present disclosure.

For example, the variant of the present disclosure may have or include an amino acid sequence of SEQ ID NO: 3 or 5, or essentially consist of the amino acid sequence of SEQ ID NO: 3 or 5. Alternatively, the variant may include an amino acid sequence having at least 70%, 75%, 80%, 85%, 90%, 95%, 96%, 97%, 98%, 99%, 99.5%, 99.7%, or 99.9% or more homology or identity with an amino acid sequence selected from the group consisting of amino acid sequences of SEQ ID NOS: 3 and 5.

For example, this includes additions or deletions of sequences to the N-terminus, C-terminus, and/or within an internal region of the amino acid sequence, naturally-occurring mutations, silent mutations, or conservative substitutions that do not alter the functions of the variant of the present disclosure.

As used herein, the term "conservative substitution" refers to the substitution of an amino acid with another amino acid having similar structural and/or chemical properties. Such amino acid substitution may generally occur based on the similarity in polarity, charge, solubility, hydrophobicity, hydrophilicity, and/or amphipathic nature of the residues. For example, positively charged (basic) amino acids include arginine, lysine, and histidine; negatively charged (acidic) amino acids include glutamate and aspartate; aromatic amino acids include phenylalanine, tryptophan, and tyrosine; and hydrophobic amino acids include alanine, valine, isoleucine, leucine, methionine, phenylalanine, tyrosine, and tryptophan. Further, amino acids may be classified into amino acids with electrically charged side chains and amino acids with uncharged side chains. The amino acids with electrically charged side chains include aspartate, glutamate, lysine, arginine, and histidine. The amino acids with uncharged side chains can be further classified into nonpolar amino acids and polar amino acids; nonpolar amino acids include glycine, alanine, valine, leucine, isoleucine, methionine, phenylalanine, tryptophan, and proline, and polar amino acids include serine, threonine, cysteine, tyrosine, asparagine, and glutamine. Typically, conserved substitution has little or no effect on the activity of the produced polypeptide. Typically, conserved substitution may have little or no effect on the activity of a protein or polypeptide.

In one embodiment, the acetohydroxy acid synthase subunit (ilvN) variant of the present disclosure may have increased acetohydroxy acid synthase subunit (ilvN) activity, but is not limited thereto. Further, the variant of the present disclosure may have the activity of increasing L-valine producing ability higher than that of a wild-type polypeptide having acetohydroxy acid synthase subunit (ilvN) activity, but is not limited thereto.

Another aspect of the present disclosure provides a polynucleotide encoding the variant of the present disclosure.

As used herein, the term "polynucleotide" refers to a polymer of nucleotides in which nucleotide monomers are chain-extended lengthwise by covalent bonds, which is a DNA or RNA strand of at least a certain length, and more specifically, a polynucleotide fragment encoding the protein.

The polynucleotide encoding the acetohydroxy acid synthase subunit (ilvN) variant of the present disclosure may comprise any sequence without limitation, provided that the sequence is a polynucleotide sequence encoding the acetohydroxy acid synthase subunit (ilvN) variant of the present disclosure. For example, the polynucleotide encoding the acetohydroxy acid synthase subunit (ilvN) variant of the present disclosure may be a polynucleotide sequence encoding the amino acid sequence of the acetohydroxy acid synthase subunit (ilvN) variant of the present disclosure, but is not limited thereto.

The polynucleotide of the present disclosure may have various modifications in the coding region within the scope that does not change the amino acid sequence of the variant of the present disclosure, in consideration of the codon degeneracy or the preferred codons of the organism in which the present disclosure is to be expressed. Accordingly, it is obvious that a polynucleotide that may be translated, by codon degeneracy, into a polypeptide consisting of an amino sequence of the variant of the present disclosure or a polypeptide having homology or identity therewith may also be included.

For example, the polynucleotide of the present disclosure may have or comprise a nucleotide sequence having 70% or more, 75% or more, 80% or more, 85% or more, 90% or more, 95% or more, 96% or more, 97% or more, or 98% or more, and less than 100% homology or identity with the sequence of SEQ ID NO: 4 or 6, or may consist of or essentially consist of a nucleotide sequence having 70% or more, 75% or more, 80% or more, 85% or more, 90% or more, 95% or more, 96% or more, 97% or more, or 98% or more, and less than 100% homology or identity with the sequence of SEQ ID NO: 4 or 6, but is not limited thereto. Alternatively, in a sequence having such homology or identity, the codon encoding the amino acid corresponding to position 159 in SEQ ID NO: 3 or SEQ ID NO: 5 may be one of the codons encoding glutamate, and the codon encoding the amino acid corresponding to position 42 in SEQ ID NO: 3 may be one of the codons encoding valine, but the codons are not limited thereto.

Further, the polynucleotide of the present disclosure may include any probe that can be prepared from a known gene sequence, for example, a sequence that can hybridize with a sequence complementary to all or part of the polynucleotide sequence of the present disclosure under stringent conditions, without limitation. The term "stringent conditions" refers to conditions that enable specific hybridization between polynucleotides. These conditions are specifically described in the literature (see J. Sambrook et al., Molecular Cloning, A Laboratory Manual, 2nd Edition, Cold Spring Harbor Laboratory press, Cold Spring Harbor, New York, 1989; and F. M. Ausubel et al., Current Protocols in Molecular Biology, John Wiley & Sons, Inc., New York, 9.50-9.51, 11.7-11.8). Examples of the stringent conditions may include conditions in which polynucleotides having high homology or identity, for example, polynucleotides having 70% or more, 75% or more, 80% or more, 85% or more, 90% or more, 95% or more, 96% or more, 97% or more, 98% or more, or 99% or more homology or identity, hybridize with each other, while polynucleotides having lower homology or identity do not hybridize with each other; or typical washing conditions for Southern hybridization, i.e., conditions of washing once, or two or three times, at a salt concentration and temperature corresponding to 1×SSC, 0.1% SDS at 60°C, specifically 0.1×SSC, 0.1% SDS at 60°C, or more specifically 0.1×SSC, 0.1% SDS at 68°C.

Hybridization requires that two nucleic acids have complementary sequences although mismatches between bases may be possible depending on the stringency of the hybridization. The term "complementary" is used to describe the relationship between nucleotide bases that are capable of hybridizing with each another. For example, with respect to DNA, adenine is complementary to thymine, and cytosine is complementary to guanine. Accordingly, the polynucleotide of the present disclosure may further include not only substantially similar nucleic acid sequences but also isolated nucleic acid fragments that are complementary to the full sequence.

Specifically, polynucleotides having homology or identity with the polynucleotide of the present disclosure may be detected by employing hybridization conditions including a hybridization step at Tₘ of 55°C and the above-described conditions. Additionally, the Tₘ value may be 60°C, 63°C, or 65°C, but is not limited thereto. The Tₘ value may be appropriately adjusted by a person of ordinary skill in the art according to the purpose.

The appropriate stringency for hybridizing the polynucleotides depends on the lengths and degree of complementarity of the polynucleotides, and the variables are well known in the art (for example, J. Sambrook *et al.,* supra).

As used herein, the term "homology" or "identity" refers to the degree of similarity between two given amino acid sequences or nucleotide sequences and may be expressed as a percentage. The terms "homology" and "identity" may often be used interchangeably.

The sequence homology or identity of conserved polynucleotides or polypeptides may be determined by the standard alignment algorithm, and default gap penalties established by the program used may be applied together. Substantially, homologous or identical sequences may generally hybridize with each other as a whole or in part under moderate or highly stringent conditions. It is obvious that the hybridization also includes hybridization with a polynucleotide containing usual codons or codons considering codon degeneracy in the polynucleotide.

Whether any two polynucleotide or polypeptide sequences have homology, similarity, or identity may be determined by using, for example, a known computer algorithm such as the "FASTA" program using default parameters as in Pearson et al. (1988) [Proc. Natl. Acad. Sci. USA 85]: 2444. Alternatively, they may be determined by using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al., Nucleic Acids Research 12: 387 (1984)), BLASTP, BLASTN, FASTA (Atschul, S. F., et al., J Molec Biol 215: 403 (1990); Guide to Huge Computers, Martin J. Bishop, ed.,] Academic Press, San Diego, 1994, and CARILLO et al.(1988) SIAM J Applied Math 48: 1073). For example, the homology, similarity, or identity may be determined by using BLAST of the National Center for Biotechnology Information or ClustalW.

The homology, similarity, or identity between polynucleotides or polypeptides may be determined by comparing sequence information using the GAP computer program as in, for example, Needleman et al., (1970), J Mol Biol. 48:443, as described in Smith and Waterman, Adv. Appl. Math (1981) 2:482. Briefly, the GAP program defines similarity as the number of similar aligned symbols (i.e., nucleotides or amino acids) divided by the total number of symbols in the shorter of the two sequences. The default parameters for the GAP program may include: (1) a binary comparison matrix (containing a value of 1 for identity and 0 for non- identity) and the weighted comparison matrix of Gribskov et al. (1986) Nucl. Acids Res. 14:6745 as disclosed in Schwartz and Dayhoff, eds., Atlas of Protein Sequence and Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps. Accordingly, the term "homology" or "identity" used herein refers to the relevance between sequences.

Another aspect of the present disclosure provides a vector comprising the polynucleotide of the present disclosure. The vector may be an expression vector for expressing the polynucleotide in a microorganism, but is not limited thereto.

As used herein, the term "vector" may include a DNA construct comprising the nucleotide sequence of a polynucleotide encoding the target polypeptide, operably linked to an appropriate expression control region (or expression control sequence) so as to express the target polypeptide in an appropriate host. The expression control region may include a promoter capable of initiating transcription, any operator sequence for controlling such transcription, a sequence encoding a suitable mRNA ribosome binding site, and sequences for controlling the termination of transcription and translation. The vector, after transformation into a suitable microorganism, may be replicated or function independently of the genome of the host, or may be integrated into the genome itself.

The vector used in the present disclosure is not particularly limited, and any vector known in the art may be used. Examples of commonly used vectors include native or recombinant plasmids, cosmids, viruses, and bacteriophages. For example, pWE15, M13, MBL3, MBL4, IXII, ASHII, APII, t10, t11, Charon4A, and Charon21A may be used as phage vectors or cosmid vectors, and pDZ-based, pBR-based, pUC-based, pBluescriptll-based, pGEM- based, pTZ-based, pCL-based, and pET-based vectors may be used as plasmid vectors. Specifically, pDZ, pDC, pDCM2, pACYC177, pACYC184, pCL, pECCG117, pUC19, pBR322, pMW118, and pCC1BAC vectors may be used.

In an embodiment, a polynucleotide encoding a target polypeptide may be inserted into a chromosome through a vector for chromosomal insertion in a cell. The insertion of the polynucleotide into the chromosome may be performed by using any method known in the art, for example, homologous recombination, but is not limited thereto. The vector may further include a selection marker for confirming the chromosomal insertion. The selection marker is used for selecting vector-transformed cells, that is, confirming the insertion a target nucleic acid molecule, and markers imparting a selectable phenotype, such as drug resistance, auxotrophy, cytotoxic drug resistance, or surface polypeptide expression, may be used. In an environment treated with selective agents, only the cells expressing the selection marker survive or exhibit a different phenotypic expression, thereby enabling the selection of the transformed cells.

As used herein, the term "transformation" means that a vector including a polynucleotide encoding a target polypeptide is introduced into a microorganism or host cell to enable expression of the polypeptide encoded by the polynucleotide in the microorganism. The transformed polynucleotide may include any polypeptide as long as it can be expressed in a microorganism, regardless of whether it is inserted and located into the chromosome of the microorganism or located outside of the chromosome. Further, the polynucleotide includes DNA and/or RNA encoding the target polypeptide. The polynucleotide may be introduced in any form as long as the polynucleotide can be introduced and expressed in a microorganism. For example, the polynucleotide may be introduced into a microorganism in the form of an expression cassette, which is a gene construct including all factors required for self-expression. The expression cassette may generally include a promoter operably linked to the polynucleotide, a transcription termination signal, a ribosome binding site, and a translation termination signal. The expression cassette may be in the form of an expression vector that is capable of self-replication. Further, the polynucleotide may be introduced into the microorganism as-is and operably linked to a sequence required for expression in the microorganism, but is not limited thereto.

Additionally, the term "operably linked" used herein refers to a functional linkage between a promoter sequence for initiating and mediating the transcription of the polynucleotide encoding the target protein of the present disclosure, and the polynucleotide sequence.

Another aspect of the present disclosure provides a microorganism comprising the acetohydroxy acid synthase subunit (ilvN) variant of the present disclosure, a polynucleotide encoding the acetohydroxy acid synthase subunit (ilvN) variant of the present disclosure, or a vector comprising the polynucleotide of the present disclosure.

In one embodiment, the microorganism of the present disclosure may be a microorganism having L-valine producing ability.

As used herein, the term "L-valine" refers to an L-amino acid having a chemical formula of (CH₃)₂CHCH(NH₂)COOH, which is an essential amino acid classified as a branched-chain amino acid along with L-leucine and L-isoleucine.

As used herein, the term "microorganism (or strain)" includes all of wild-type microorganisms and microorganisms with natural or artificial genetic modification and refers to a microorganism in which a particular mechanism is weakened or enhanced due to the insertion of an exogenous gene, the enhancement or inactivation of the activity of an endogenous gene, or the like. The microorganism may be a microorganism including a genetic modification for the production of a target polypeptide, protein, or product. As used herein, the terms "microorganism", "strain", and "microbe" have the same meaning and may be used interchangeably without limitation.

As used herein, the term "L-valine-producing microorganism" refers to a prokaryotic or eukaryotic microbial strain capable of producing L-valine within its cellular system, and may include any microorganism in which L-valine producing ability has been imparted to a parent strain lacking such ability or any microorganism that inherently possesses L-valine producing ability. The L-valine producing ability may be imparted or enhanced by strain improvement.

In one embodiment, the microorganism of the present disclosure may be a microorganism naturally possessing an acetohydroxy acid synthase subunit (ilvN) variant or having L-valine producing ability; or a microorganism in which the variant of the present disclosure or a polynucleotide encoding thereof (or a vector comprising the polynucleotide) is introduced and/or L-valine producing ability is imparted to a parent strain with no acetohydroxy acid synthase subunit (ilvN) variant or L-valine producing ability, but is not limited thereto.

In one embodiment, the microorganism of the present disclosure includes any microorganism comprising the sequence of the acetohydroxy acid synthase subunit (ilvN) variant of the present disclosure by mutations in the chromosomal gene encoding the acetohydroxy acid synthase subunit (ilvN) variant and/or any microorganism comprising the acetohydroxy acid synthase subunit (ilvN) variant of the present disclosure by introducing a vector comprising a polynucleotide encoding the acetohydroxy acid synthase subunit (ilvN) variant of the present disclosure, but is not limited thereto.

As used herein, the term "non-modified microorganism" does not exclude strains including mutations that may naturally occur in microorganisms and may refer to a wild-type strain or native strain as-is, or a strain before its trait is changed due to a genetic mutation caused by natural or artificial factors. For example, the non-modified microorganism may refer to a strain in which the acetohydroxy acid synthase subunit (ilvN) variant of the present disclosure is not introduced or a strain before its introduction. The "non-modified microorganism" may be used interchangeably with "strain before modification", "microorganism before modification", "non-mutated strain", "non-modified strain", "non-mutated microorganism", or "reference microorganism".

**The** microorganism having L-valine producing ability of the present disclosure may be a microorganism comprising one or more selected from the variant of the present disclosure, the polynucleotide of the present disclosure, and a vector comprising the polynucleotide of the present disclosure; a microorganism modified to express the variant of the present disclosure or the polynucleotide of the present disclosure; a microorganism (*e.g*., recombinant strain) expressing the variant of the present disclosure or the polynucleotide of the present disclosure; or a microorganism (*e.g*., recombinant strain) having the activity of the variant of the present disclosure, but is not limited thereto.

For example, the strain of the present disclosure refers to a cell or microorganism expressing the variant of the present disclosure by transforming into a vector comprising the polynucleotide of the present disclosure or polynucleotide encoding the variant of the present disclosure, and the strain of the present disclosure may comprise any microorganism capable of producing L-valine by comprising the variant of the present disclosure. For example, the microorganism of the present disclosure may be a recombinant strain with increased L-valine producing ability, in which the increased L-valine producing ability is achieved by introducing a polynucleotide encoding the variant of the present disclosure into a natural wild-type microorganism or microorganism having L-valine producing ability and thereby enabling the expression of an acetohydroxy acid synthase subunit (ilvN) variant. The recombinant strain with increased L-valine producing ability may be a microorganism with increased L-valine producing ability compared with a natural wild-type microorganism or a microorganism with non-modified acetohydroxy acid synthase subunit (ilvN) (e.g., a microorganism expressing a wild-type acetohydroxy acid synthase subunit (ilvN) or a microorganism that does not express the variant of the present disclosure), but is not limited thereto. For example, the microorganism with increased L-valine producing ability of the present disclosure may be a microorganism with increased L-valine producing ability compared with a microorganism comprising the polypeptide of SEQ ID NO: 1 or a polynucleotide encoding thereof, but is not limited thereto.

The microorganism of the present disclosure may include any microorganism capable of expressing the acetohydroxy acid synthase subunit (ilvN) variant of the present disclosure through various known methods, in addition to the introduction of nucleic acid or vector.

For example, the microorganism with increased L-valine producing ability may have L-valine producing ability increased by about 1% or more, specifically, about 1% or more, about 2.5% or more, about 5% or more, about 6% or more, about 7% or more, about 8% or more, about 9% or more, about 10% or more, about 10.5% or more, about 11% or more, about 11.5% or more, about 12% or more, about 12.5% or more, about 13% or more, about 13.5% or more, about 14% or more, about 14.5% or more, about 14.6% or more, about 14.7% or more, or about 14.8% or more (the upper limit is not particularly limited and may be, for example, about 200% or less, about 150% or less, about 100% or less, about 50% or less, about 45% or less, about 40% or less, about 35% or less, about 30% or less, about 25% or less, about 20% or less, or about 15% or less) compared with the L-valine producing ability of the parent strain before modification or non-modified microorganism. However, as long as the microorganism shows a positive increase in L-valine producing ability compared with the parent strain or non-modified microorganism, the L-valine producing ability is not limited thereto. In another example, the recombinant strain with increased L-valine producing ability may have L-valine producing ability increased about 1.1 times or more, about 1.12 times or more, about 1.13 times or more, or about 1.14 times or more (the upper limit is not particularly limited and may be, for example, about 10 times or less, about 5 times or less, about 3 times or less, about 2 times or less, about 1.5 times or less, or about 1.2 times or less) compared with the L-valine producing ability of the parent strain before modification or a non-modified microorganism, but the L-valine producing ability is not limited thereto. As used herein, the term "about" refers to a range encompassing all of ±0.5, ±0.4, ±0.3, ±0.2, ±0.1, and the like, which encompasses all of the values in the range equivalent or similar to those stated after the term "about", but is not limited thereto.

The microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a microorganism of the genus *Corynebacterium,* or more specifically *Corynebacterium glutamicum,* but is not limited thereto.

Meanwhile, it is already known that microorganisms of the genus *Corynebacterium* are capable of producing L-valine; however, their production capacity is significantly low, and the genes involved in the production mechanism and the underlying principles of the mechanism have not yet been fully elucidated. Accordingly, the microorganism of the genus *Corynebacterium* having L-valine producing ability of the present disclosure includes any microorganism of the genus *Corynebacterium* with increased L-valine producing ability by enhancing or weakening the activity of a gene related to the L-valine production mechanism or microorganism of the genus *Corynebacterium* with increased L-valine producing ability by introducing or enhancing the activity of an exogenous gene.

Further, the microorganism of the present disclosure may have enhanced activity of the acetohydroxy acid synthase subunit (ilvN) compared with the parent strain.

As used herein, the term "enhancement" of polypeptide activity means that the polypeptide activity is increased, as compared with the intrinsic activity. The enhancement may be used interchangeably with terms such as "activation", "up-regulation", "overexpression", and "increase". As used herein, the terms "activation", "enhancement", "up-regulation", "overexpression", and "increase" may include both of the exhibition of an activity that was not originally possessed and the exhibition of improved activity as compared with the intrinsic activity or activity before modification. The term "intrinsic activity" refers to the activity of a specific polypeptide originally possessed by the parent strain before the trait is changed or an unmodified microorganism when the trait is changed by genetic variation due to natural or artificial factors. This term may be used interchangeably with "activity before modification". The "enhancement", "up-regulation", "overexpression", or "increase" of a polypeptide activity compared with its intrinsic activity refers to the increase in activity and/or concentration (expression level) of a specific polypeptide naturally possessed by the parent strain before transformation or nonmodified microorganism.

The enhancement of the activity of the polypeptide could be achieved by applying various methods well known in the art. The methods include, for example, increasing the intracellular copy number of the gene encoding the variant, introducing a mutation into a gene expression control sequence on the chromosome encoding the variant, replacing the gene expression control sequence on the chromosome encoding the variant with a sequence with enhanced activity, substituting the chromosomal gene encoding the protein with a gene mutated to have increased activity of the variant, and introducing a mutation to a chromosomal gene encoding the variant protein so as to enhance the activity of the variant, but are not limited thereto.

As used herein, the term "introduction" refers to the delivery of a polynucleotide encoding the acetohydroxy acid synthase variant or a vector comprising thereof into a host cell. Such introduction can be readily achieved using common methods in the art. The common methods include CaCl₂ precipitation, the Hanahan method using the reducing agent dimethyl sulfoxide (DMSO) to the CaCl₂ method to increase the efficiency, electroporation, calcium phosphate precipitation, protoplast fusion, stirring using silicon carbide fibers, transformation using PEG, and transformation mediated by dextran sulfate, Lipofectamine, or drying/inhibition. Methods for transforming the vector are not limited to these examples, and any transformation or transfection methods commonly used in the art may be used without limitation. Additionally, the delivered polynucleotide may be introduced and located within the chromosome of the host cell or be located outside the chromosome, as long as it can be expressed within the host cell. Furthermore, the polynucleotide may be introduced into the host cell in any form, as long as it can be expressed within the host cell. For example, the nucleotide may be introduced into the host cell in the form of an expression cassette, which is a polynucleotide construct comprising all the necessary elements for self-expression, but the form is not limited thereto. The expression cassette generally includes a promoter operably linked to the open reading frame (hereinafter referred to as "ORF") of the gene, a transcription termination signal, a ribosome binding site, and a translation termination signal. The expression cassette may be in the form of an expression vector that is capable of self-replication. Further, the polynucleotide may be introduced into the host cell as-is and be operably linked to the sequence necessary for expression in the host cell, but is not particularly limited thereto.

Another aspect of the present disclosure provides a method for producing L-valine, comprising culturing the microorganism of the present disclosure in a medium.

Specifically, the method for producing L-valine of the present disclosure may comprise culturing a microorganism comprising the variant of the present disclosure, the polynucleotide of the present disclosure, or the vector of the present disclosure in a medium, but is not limited thereto.

As used herein, the term "culturing" refers to growing the microorganism of the present disclosure under appropriately controlled environmental conditions. The culturing process of the present disclosure may be performed according to suitable medium and culture conditions known in the art. Such a culture process may be easily adjusted and used by those skilled in the art according to the selected strain. Specifically, the culturing may be a batch type, continuous type, and/or fed-batch type, but is not limited thereto.

As used herein, the term "medium" means a mixed substance containing nutrients required to culture the microorganism of the present disclosure as a main component, and the medium supplies nutrients, growth factors, *etc.,* including water, which are indispensable for survival and development. Specifically, any medium and culture conditions may be used for culturing the microorganism of the present disclosure without particular limitation, as long as the medium is commonly used for the culture of microorganisms. The microorganism of the present disclosure may be cultured in a common medium containing suitable carbon sources, nitrogen sources, phosphorus sources, inorganic compounds, amino acids and/or vitamins, *etc.,* while controlling the temperature, pH, *etc.* under aerobic conditions.

For example, a culture medium for the strain of the genus *Corynebacterium* can be found in the document ["Manual of Methods for General Bacteriology" by the American Society for Bacteriology (Washington D.C., USA, 1981)].

In the present disclosure, the carbon sources include carbohydrates such as glucose, saccharose, lactose, fructose, sucrose, maltose, *etc.;* sugar alcohols such as mannitol, sorbitol, *etc.,* organic acids such as pyruvic acid, lactic acid, citric acid, *etc.;* and amino acids such as glutamate, methionine, lysine, *etc.* Further, natural organic nutrients such as starch hydrolysates, molasses, blackstrap molasses, rice bran, cassava, sugarcane residues, and corn steep liquor may be used. Specifically, carbohydrates such as glucose and sterilized pretreated molasses (*i.e*., molasses converted to reducing sugars) may be used, and appropriate amounts of other carbon sources may be used in various manners without limitation. These carbon sources may be used singly or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, ammonium nitrate, *etc.;* and organic nitrogen sources such as amino acids such as glutamic acid, methionine, glutamine, *etc.,* peptone, NZ-amine, meat extracts, yeast extract, malt extract, corn steep liquor, casein hydrolysate, fish or decomposition products thereof, and defatted soybean cake or decomposition products thereof may be used. These nitrogen sources may be used singly or in combination of two or more thereof, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, *etc.* may be used. In addition, amino acids, vitamins and/or suitable precursors, *etc.* may be included. These components or precursors may be added to the medium in a batchwise or continuous manner, but the methods are not limited thereto.

Further, during the culturing of the microorganism of the present disclosure, the pH of the medium may be adjusted by adding compounds such as ammonium hydroxide, potassium hydroxide, ammonia, phosphoric acid, sulfuric acid, *etc.* to the medium in a suitable manner. Further, during the culturing, foaming may be suppressed by using an antifoaming agent such as fatty acid polyglycol ester. Further, oxygen or oxygen-containing gas may be injected into the medium in order to maintain the aerobic state of the medium, or nitrogen, hydrogen, or carbon dioxide gas, or no gas may be injected to maintain the anaerobic and microaerobic states, but the method is not limited thereto.

In the culturing of the present disclosure, the culture temperature may be maintained at 20°C to 45°C, specifically, at 25°C to 40°C, and the strain may be cultured for about 10 to 160 hours, but the culture conditions are not limited thereto.

L-valine produced according to the culturing of the present disclosure may be secreted into the medium or remain within the cell.

The method for producing L-valine of the present disclosure may further comprise preparing the microorganism of the present disclosure, preparing a medium for culturing the strain, or a combination thereof in any order, for example, before culturing.

The method for producing L-valine of the present disclosure may further comprise recovering L-valine from the medium of the culturing (the medium in which culturing has been performed) or from the microorganism of the present disclosure. The method may further comprise the recovering after the culturing.

The recovering may be collecting L-valine using suitable methods known in the art depending on the culturing method of the microorganism of the present disclosure, such as batch, continuous, or fed-batch culturing methods. For example, L-valine can be recovered from the medium or microorganism using suitable methods known in the art, such as centrifugation, filtration, crystallization, treatment with protein precipitants (salting-out method), extraction, ultrasonic disruption, ultrafiltration, dialysis, various types of chromatography such as molecular sieve chromatography (gel filtration), adsorption chromatography, ion exchange chromatography, and affinity chromatography, HPLC, or combinations thereof.

Further, the method for producing L-valine of the present disclosure may further comprise purification. The purification may be performed using suitable methods known in the art. For example, when the method for producing L-valine of the present disclosure comprises both the recovering and purification, the recovering and purification may be performed sequentially or non-sequentially, in any order, or they may be carried out simultaneously or integrated into a single step; however, the method is not limited thereto.

Another aspect of the present disclosure provides a composition for producing L-valine, comprising the acetohydroxy acid synthase subunit (ilvN) variant of the present disclosure, a polynucleotide encoding the variant of the present disclosure, a vector comprising the polynucleotide of the present disclosure, or a microorganism comprising the polynucleotide of the present disclosure; medium obtained by culturing the microorganism; or a combination of two or more thereof.

The composition of the present disclosure may further comprise any suitable excipients commonly used in compositions for amino acid production. Such excipients include, for example, preservatives, humectants, dispersants, suspending agents, buffers, stabilizers, or isotonic agents, but are not limited thereto.

### [Mode for Carrying Out the Invention]

The present disclosure will be described in detail by way of Examples. However, these Examples are given for illustrative purposes only, and the scope of the invention is not intended to be limited by these Examples. Meanwhile, the technical descriptions absent in the present disclosure may be sufficiently understood and readily practiced by a person of ordinary skill in the art of the present disclosure or related art.

### Example 1. Selection of mutant strains with increased valine producing ability through artificial mutagenesis

### Example 1-1. Artificial mutagenesis through UV exposure

In order to select the mutant strains with increased valine producing ability, *Corynebacterium glutamicum* KCCM11201P (Korean Patent No. 10-1117022), a valine-producing strain, was inoculated onto a nutrient medium containing agar and cultured at 30°C for 36 hours. Hundreds of colonies thus obtained were irradiated with UV at room temperature to induce random mutagenesis in the genome of the strain.

### Example 1-2. Evaluation of fermentation potency of mutagenized strains and strain selection

In order to select mutant strains with increased L-valine producing ability compared with *Corynebacterium glutamicum* KCCM11201P, which was used as the parent strain, a fermentation potency test was performed on the randomly mutagenized strains. After subculturing in a nutrient medium, each strain was inoculated into a 250 mL corner-baffle flask containing 25 mL of a production medium and incubated with shaking at 200 rpm at 30°C for 72 hours. The concentration of L-valine was analyzed using HPLC, and the analyzed concentrations of L-valine were tabulated in Table 1.

### [Nutrient Medium (pH 7.2)]

glucose 10 g, meat extract 5 g, polypeptone 10 g, sodium chloride 2.5 g, yeast extract 5 g, agar 20 g, and urea 2 g (based on 1 L of distilled water)

### [Production Medium (pH 7.0)]

glucose 100 g, ammonium sulfate 40 g, soy protein 2.5 g, corn steep solids 5 g, urea 3 g, potassium phosphate dibasic 1 g, magnesium sulfate heptahydrate 0.5 g, biotin 100 µg, thiamine-HCl 1 mg, calcium pantothenate 2 mg, nicotine amide 3 mg, calcium carbonate 30 g (based on 1 L of distilled water)

**[Table 1]**

| | Name of Strain | L-valine (g/L) |
|---|---|---|
| Control group | KCCM11201P | 2.7 |
| Experimental group | C1 | 1.7 |
| | C2 | 1.7 |
| | C3 | 0.5 |
| | C4 | 2.5 |
| | C5 | 1.9 |
| | C6 | 3.1 |
| | C7 | 2.2 |

Based on the results shown in Table 1, C6 strain which showed the highest increase in valine production compared with the KCCM11201P strain (i.e., the control), was selected.

### Example 2. Identification of mutation through gene sequencing

The main genes of the strain were sequenced and compared with those of the KCCM11201P strain. As a result, the C6 strain with increased valine producing ability was found to comprise nucleotide sequence mutations at specific positions of the ORF region of the *ilvN* gene.

Specifically, it was found that the C6 strain, which showed the highest increase in valine production, identically included the A42V mutation in the parent strain KCCM11201P, along with a mutation introduced 476 bp upstream from the start codon of the *ilvN* gene. This mutation changed the original codon GCA (SEQ ID NO: 2) to GAA (SEQ ID NO: 4), resulting in the substitution of the amino acid at position 159 (alanine) with glutamate (SEQ ID NO: 3).

Upon analyzing the A159E mutation region, the mutation was found to affect the effector binding domain of the valine biosynthetic enzyme, and thus it was expected that the activity of the corresponding protein would be enhanced. Hereinafter, the following Examples identify whether the application of the A159E mutation inserted at a specific position in the *ilvN* gene would affect the ability to produce valine, a branched-chain amino acid, of the microorganism of the genus *Corynebacterium.*

### Example 3. Preparation of KCCM11201P strains introduced with ilvN mutation and identification of valine producing ability

### Example 3-1. Preparation of Corynebacterium glutamicum KCCM11201P strains introduced with ilvN mutations and evaluation of L-valine producing ability - 1

To insert an ilvN (A159E) mutation into a *Corynebacterium glutamicum* KCCM11201P strain with A42V mutation, a vector containing the target mutation was constructed. Specifically, the genomic DNA of the C6 strain was extracted using a G-spin Total DNA Extraction Mini Kit (Intron, Cat. No. 17045) according to the protocol provided in the kit, and PCR was performed using the genomic DNA as a template. The conditions for PCR were as follows: denaturation at 94°C for 5 minutes; 25 cycles of denaturation at 94°C for 30 seconds, annealing at 55°C for 30 seconds, and polymerization at 72°C for 150 seconds; and polymerization at 72°C for 7 minutes. A 1010 bp PCR product (hereinafter referred to as "mutation-introduced fragment 1") was obtained using SEQ ID NOS: 7 and 8.

The obtained mutation-introduced fragment 1 was ligated to a pDCM2 vector (Korean Patent Publication No. 10-2020-136813; International Patent Publication No. 2008-033001) treated with the restriction enzyme *Xba*l (New England Biolabs, Beverly, MA) using an Infusion Cloning Kit (Takara Bio Inc., Otsu, Japan), followed by transformation into E. *coli* DH5α. After transforming the prepared gene into E. *coli* DH5α, the transformed strains were selected in an LB medium containing kanamycin. DNA was obtained therefrom using a DNA-spin plasmid DNA purification kit (INTRON), which was used to construct a pDCM2-ilvN(A42V, A159E) vector comprising the mutation-introduced fragment 1.

**[Table 2]**

| Primer | Nucleotide Sequence |
|---|---|
| SEQ ID NO: 7 | cggggatcctctaga AGGACGGTACTCAAATACTAAACTTC |
| SEQ ID NO: 8 | cggggatcctctaga GACAACTACATTATTATTATACCACA |

The pDCM2-ilvN(A42V, A159E) vector was transformed into *Corynebacterium glutamicum* KCCM11201 P by homologous recombination on the chromosome (van der Rest et al., Appl Microbiol Biotechnol 52:541-545, 1999). Strains in which the vector is inserted into the chromosome by homologous sequence recombination were selected in a medium containing kanamycin (25 mg/L). A PCR using SEQ ID NOS: 7 and 8 was performed on the *Corynebacterium glutamicum* transformants that had undergone secondary recombination to identify a strain which identically includes A42V mutation (i.e., alanine was substituted with valine at position 42 in the amino acid sequence of SEQ ID NO: 1 within the ORF of the *ilvN* gene on the chromosome), which is included in the parent strain KCCM11201P, in addition to the substitution of alanine with glutamate at position 159 in the amino acid sequence of SEQ ID NO: 1 within ORF of the *ilvN* gene on the chromosome. The recombinant strain was named *Corynebacterium glutamicum* KCCM11201P::*ilvN*(A159E)*.*

In order to compare the valine producing ability of the valine-producing strains *Corynebacterium glutamicum* KCCM11201P and KCCM11201P*::ilvN*(A159E), flask evaluation was performed. Each strain was subcultured in a nutrient medium, inoculated into a 250 mL corner-baffled flask containing 25 mL of a production medium, and incubated with shaking at 200 rpm at 30°C for 72 hours. The concentration of L-valine was analyzed using HPLC, and the analyzed concentrations of L-valine are shown in Table 3.

### [Nutrient Medium (pH 7.2)]

glucose 10 g, meat juice 5 g, polypeptone 10 g, sodium chloride 2.5 g, yeast extract 5 g, agar 20 g, and urea 2 g (based on 1 L of distilled water)

### [Production Medium (pH 7.0)]

glucose 100 g, ammonium sulfate 40 g, soy protein 2.5 g, corn steep solids 5 g, urea 3 g, potassium phosphate dibasic 1 g, magnesium sulfate heptahydrate 0.5 g, biotin 100 µg, thiamine-HCl 1 mg, calcium pantothenate 2 mg, nicotine amide 3 mg, calcium carbonate 30 g (based on 1 L of distilled water)

**[Table 3]**

| KCCM11201P and KCCM11201P::ilvN(A42V+A159E), | | | | |
|---|---|---|---|---|
| Strains with L-valine producing ability | L-valine (g/L) | | | |
| | Batch 1 | Batch 2 | Batch 3 | Mean |
| KCCM11201 P | 2.7 | 2.7 | 2.7 | 2.7 |
| KCCM11201P ::*ilvN*(A159E) | 3.1 | 3.1 | 3.2 | 3.1 |

As a result, it was found that the KCCM11201P::ilvN(A159E) strain showed an increase in L-valine producing ability by 14.8% compared with the KCCM11201P strain.

### Example 3-2. Preparation of Corynebacterium glutamicum KCCM11201P strains introduced with ilvN mutations and evaluation of L-valine producing ability -2

In order to identify the effect of the ilvN(A159E) mutation alone in the wild-type ilvN enzyme, a strain was constructed in which the amino acid (alanine) at position 159 of SEQ ID NO: 1 in the ORF region of the *ilvN* gene is substituted with glutamate. Specifically, a vector comprising the target mutation was constructed to insert ilvN(V42A, A159E) into the *Corynebacterium glutamicum* KCCM11201P strain. Specifically, the genomic DNA of the C6 strain was extracted using a G-spin Total DNA Extraction Mini Kit (Intron, Cat. No. 17045) according to the protocol provided in the kit, and PCR was performed using the genomic DNA as a template. The conditions for PCR were as follows: denaturation at 94°C for 5 minutes; 25 cycles of denaturation at 94°C for 30 seconds, annealing at 55°C for 30 seconds, and polymerization at 72°C for 150 seconds; and then polymerization at 72°C for 7 minutes. A 515 bp PCR product (hereinafter referred to as "mutation-introduced fragment 2") and a 518 bp PCR product (hereinafter referred to as "mutation-introduced fragment 3") were each obtained using SEQ ID NOS: 9 and 10, and SEQ ID NOS: 11 and 12, respectively.

The obtained mutation-introduced fragments 2 and 3 were ligated to a pDCM2 vector (Korean Patent Application Publication No. 10-2020-136813; International Patent Publication No. 2008-033001) treated with the restriction enzyme *Xba*l (New England Biolabs, Beverly, MA) by using an Infusion Cloning Kit (Takara Bio Inc., Otsu, Japan), followed by transformation into *E*. *coli* DH5α. After transforming the prepared gene into *E. coli* DH5α, the transformed strains were selected in an LB medium containing kanamycin. DNA was obtained therefrom using a DNA-spin plasmid DNA purification kit (INTRON), which was used to construct a pDCM2-ilvN(V42A, A159E) vector comprising the mutation-introduced fragments 2 and 3.

**[Table 4]**

| Primer | Nucleotide Sequence |
|---|---|
| SEQ ID NO: 9 | cggggatcctctaga AGGACGGTACTCAAATACTAAACTTC |
| SEQ ID NO: 10 | GGTCTTTGCAGACACGAGGGACACGAGG |
| SEQ ID NO: 11 | TGTCCCTCGTGTCTGCAAAGACCGAAACACTCGGC |
| SEQ ID NO: 12 | cggggatcctctaga GACAACTACATTATTATTATACCACA |

The pDCM2-ilvN(V42A, A159E) vector was transformed into *Corynebacterium glutamicum* KCCM11201 P by homologous recombination on the chromosome (van der Rest et al., Appl Microbiol Biotechnol 52:541-545, 1999). Through the homologous recombination, the A42V mutation was reverted, and the A159E mutation was introduced. Strains in which the vector is inserted into the chromosome by homologous sequence recombination were selected in a medium containing kanamycin (25 mg/L). A PCR using SEQ ID NOS: 9 and 12 was performed on the *Corynebacterium glutamicum* transformants that had undergone secondary recombination to identify a strain in which valine at position 42 in the amino acid sequence of SEQ ID NO: 1 within the ORF of the *ilvN* gene on the chromosome is reverted to alanine and alanine at position 159 in the amino acid sequence is substituted with glutamic acid. The recombinant strain was named *Corynebacterium glutamicum* KCCM11201P*::ilvN*(V42A*,* A159E).

In order to compare the L-valine producing ability of the prepared strains, the strains were cultured in the same manner as in Example 3-1, and the concentration of L-valine was analyzed. The analyzed concentrations of L-valine are shown in Table 5 below.

**[Table 5]**

| KCCM11201 P, KCCM11201P::*ilvN*(V42A*,* A159E), and KCCM11201P::*ilvN*(A159E)*,* | | | | |
|---|---|---|---|---|
| Strains with L-valine producing ability | L-valine (g/L) | | | |
| | Batch 1 | Batch 2 | Batch 3 | Mean |
| KCCM11201P (ilvN A42V) | 2.7 | 2.7 | 2.7 | 2.7 |
| KCCM11201P ::*ilvN*(V42A*,* A159E) | 2.8 | 2.7 | 2.9 | 2.8 |
| KCCM11201P ::*ilvN*(A42V, A159E) | 3.1 | 3.1 | 3.2 | 3.1 |

As a result, the strains KCCM11201P::*ilvN*(V42A*,* A159E) and KCCM11201*P::iIvN(A159E)* showed an increase of 3.7% and 14.8% in the L-valine producing ability, respectively, compared with the KCCM11201P strain.

As set forth above, a person skilled in the art will be able to understand that the present disclosure may be embodied in other specific forms without departing from the technical spirit or essential characteristics thereof. Therefore, the embodiments described above should be construed as being exemplified and not limiting the present disclosure. It should be understood that all changes or modifications derived from the definitions and the scopes of the claims and their equivalents fall within the scope of the present disclosure.

## Claims

1. An acetohydroxy acid synthase subunit (ilvN) variant, wherein an amino acid corresponding to position 159 in an amino acid sequence of SEQ ID NO: 1 is substituted with another amino acid.

2. The variant of claim 1, wherein an amino acid corresponding to position 42 in the amino acid sequence of SEQ ID NO: 1 is further substituted with another amino acid.

3. The variant of claim 1, wherein an amino acid corresponding to position 159 in the amino acid sequence of SEQ ID NO: 1 is substituted with glutamate.

4. The variant of claim 2, wherein an amino acid corresponding to position 159 in the amino acid sequence of SEQ ID NO: 1 is substituted with glutamate and wherein an amino acid corresponding to position 42 in the amino acid sequence is substituted with valine.

5. The variant of claim 1, wherein the variant consists of an amino acid sequence of SEQ ID NO: 3.

6. The variant of claim 2, wherein the variant consists of an amino acid sequence of SEQ ID NO: 5.

7. A polynucleotide encoding the variant of any one of claims 1 to 6.

8. A microorganism comprising the variant of any one of claims 1 to 6 or a polynucleotide encoding the variant.

9. The microorganism of claim 8, wherein the microorganism has an increased L-valine-producing ability compared with a microorganism comprising a polypeptide of SEQ ID NO: 1 or a polynucleotide encoding the same.

10. The microorganism of claim 8, wherein the microorganism is a microorganism of the genus *Corynebacterium.*

11. The microorganism of claim 10, wherein the microorganism of the genus *Corynebacterium* is *Corynebacterium glutamicum.*

12. A method for producing L-valine, comprising culturing the microorganism of claim 8 in a medium.

13. The method of claim 12, wherein the method further comprises recovering a target substance from the medium.
